# EUROPÄISCHE PATENTSCHRIFT

(11) **EP 2 373 616 B1**
(45) Veröffentlichungstag und Bekanntmachung des Hinweises auf die Patenterteilung: **22.10.2014**
(21) Anmeldenummer: 09763939.7
(22) Anmeldetag: 30.11.2009
(51) Int. Cl.: C07C 263/10

(54) **VERFAHREN ZUR HERSTELLUNG VON ISOCYANATEN**
METHOD FOR PRODUCING ISOCYANATES
PROCÉDÉ DE PRODUCTION D'ISOCYANATES

(30) Priorität: 03.12.2008 EP 08170576
(43) Veröffentlichungstag der Anmeldung: 12.10.2011
(73) Patentinhaber: BASF SE, 67056 Ludwigshafen (DE)
(72) Erfinder: KNÖSCHE, Carsten, 67150 Niederkirchen (DE); MATTKE, Torsten, 67251 Freinsheim (DE); MÜLLER, Patric, 67112 Mutterstadt (DE)
(86) Internationale Anmeldenummer: PCT/EP2009/066023
(87) Internationale Veröffentlichungsnummer: WO 2010/063665

(56) Entgegenhaltungen:
- EP-A1- 1 403 248
- EP-A1- 1 935 875
- WO-A1-2005/123665

## Beschreibung

Die Erfindung betrifft ein Verfahren zur Herstellung von Isocyanaten durch Umsetzung der korrespondierenden Amine mit Phosgen in der Gasphase, gegebenenfalls in Gegenwart eines Inertmediums, bei dem das Amin und das Phosgen zunächst gemischt werden und in einem Reaktor zum Isocyanat umgesetzt werden. Ein den Reaktor verlassendes, Isocyanat und Chlorwasserstoff enthaltendes Reaktionsgas wird in einem Quench durch Zugabe eines flüssigen Quenchmediums abgekühlt, wobei ein Gemisch aus Reaktionsgas und Quenchmedium als Produktstrom entsteht.

Die Herstellung von Isocyanaten durch Phosgenierung der korrespondierenden Amine kann prinzipiell durch eine Flüssigphasen- oder eine Gasphasenphosgenierung erfolgen. Die Gasphasenphosgenierung zeichnet sich dadurch aus, dass eine höhere Selektivität, ein geringerer Hold-up an toxischem Phosgen sowie ein verminderter Energiebedarf erforderlich sind.

Bei der Gasphasenphosgenierung werden ein aminhaltiger Eduktstrom und ein phosgenhaltiger Eduktstrom jeweils in gasförmigem Zustand vermischt. Das Amin und das Phosgen setzen sich unter Freisetzung von Chlorwasserstoff (HCl) zu den entsprechenden Isocyanaten um. Der aminhaltige Eduktstrom liegt im Allgemeinen in flüssiger Phase vor und muss vor der Vermischung mit dem phosgenhaltigen Strom verdampft und gegebenenfalls überhitzt werden.

Entsprechende Verfahren zur Herstellung von Isocyanaten in der Gasphase sind zum Beispiel in EP-A 1 319 655 oder EP-A 1 555 258 beschrieben.

Um Folgereaktionen zu vermeiden, ist es notwendig, das Reaktionsgemisch nach Reaktionsende schnell abzukühlen. Hierzu wird zum Beispiel ein Flüssigkeitsquench eingesetzt. Ein solcher Flüssigkeitsquench ist beispielsweise in EP-A 1 403 248 oder in DE-A 10 2006 058 634 beschrieben. Das Quenchmedium, das zur Kühlung zugegeben wird, weist dabei eine Temperatur auf, die im Bereich von 50 bis 200°C liegt. Durch den eingedüsten Flüssigkeitsstrom kühlt das Reaktionsgas schnell auf Temperaturen im Allgemeinen zwischen 100 und 200°C ab. Dabei entsteht ein Zwei-Phasen-Gemisch mit einer isocyanatreichen Flüssigphase und einer isocyanatarmen Gasphase. Beide werden anschließend einer gemeinsamen oder gegebenenfalls separaten Trennstufe, zum Beispiel eine Destillationsstufe zur Trennung von Chlorwasserstoff und Phosgen auf der einen Seite und Isocyanat eventuell mit Lösungsmittel auf der anderen Seite zugeführt.

Ein Verfahren, bei dem im Quenchmedium auch das hergestellte Isocyanat enthalten ist, ist in EP-A 1 935 875 beschrieben. Durch den Isocyanat-Gehalt in dem Quenchmedium lässt sich eine höhere Isocyanat-Konzentration im den Quench verlassenden und später aufzureinigenden Produktstrom erzielen. Nachteil dieses Verfahrens, bei dem Isocyanat in dem Quenchmedium enthalten ist, ist jedoch, dass Feststoffe durch Degradation der Ausgangsstoffe und/oder der Reaktionsprodukte entstehen können, die in die nachfolgenden Anlagenteile verschleppt werden. Die Feststoffe sammeln sich in den Hochsiederströmen an und werden im Allgemeinen über Kolonnensümpfe ausgeschleust. Da jedoch aus den Hochsiederströmen Flüssigkeit als Quenchmedium entnommen wird, können im Quenchmedium Feststoffpartikel enthalten sein. Diese können in den nachfolgenden Anlagenteilen zu Ablagerungen führen. Durch die feststoffartigen Bestandteile und die Ablagerungen können Rohrleitungen, Regeleinrichtungen und andere Apparateteile verstopfen. Insbesondere können die Zerstäubungsdüsen des Quenchs verstopfen. Dies erfordert eine aufwendige Reinigung der Anlage.

Aufgabe der vorliegenden Erfindung ist es, ein Verfahren zur Herstellung von Isocyanaten durch Umsetzung der korrespondierenden Amine mit Phosgen bereitzustellen, bei dem das Quenchmedium zumindest teilweise nach Aufarbeitung in den Prozeß zurückgeführt wird und gegebenenfalls Isocyanat enthalten kann, wobei eine höhere Standzeit erreicht wird als bei den aus dem Stand der Technik bekannten Verfahren.

Gelöst wird die Aufgabe durch ein Verfahren zur Herstellung von Isocyanaten durch Umsetzung der korrespondierenden Amine mit Phosgen in der Gasphase, gegebenenfalls in Gegenwart eines Inertmediums, bei dem das Amin und das Phosgen zunächst gemischt werden und in einem Reaktor zum Isocyanat umgesetzt werden, wobei ein den Reaktor verlassendes, Isocyanat und Chlorwasserstoff enthaltendes Reaktionsgas in einem Quench durch Zugabe eines flüssigen Quenchmediums abgekühlt wird, wobei ein Gemisch aus Reaktionsgas und Quenchmedium als Produktstrom entsteht. Als Quenchmedium wird ein Gemisch eingesetzt, das mindestens Lösungsmittel oder Isocyanat enthält und das einem der Reaktion nachgeschalteten Aufarbeitungsverfahren entnommen wird, wobei in dem Quenchmedium gegebenenfalls enthaltene Feststoffpartikel vor Zugabe in den Quench entfernt werden.

Durch das Entfernen der gegebenenfalls in dem Quenchmedium enthaltenen Feststoffpartikel vor Zugabe des Quenchmediums in den Quench wird vermieden, dass sich in dem Quenchmedium enthaltene Feststoffpartikel in den Düsen ablagern und so zu einem Verstopfen der Düsen führen. Hierdurch kann die Standzeit des Quenches gegenüber den aus dem Stand der Technik bekannten Verfahren erhöht werden.

Zur Herstellung des Isocyanates werden das Phosgen und das Amin vorzugsweise zunächst einer Mischzone zugeführt, in der die Vermischung von Amin und Phosgen zu einem Reaktionsgemisch erfolgt. Anschließend wird das Reaktionsgemisch dem Reaktor zugeführt, in dem die Umsetzung zum Isocyanat erfolgt. Die Umsetzung von Amin und Phosgen im Reaktor erfolgt dabei vorzugsweise in der Gasphase. Hierzu liegt der Druck im Reaktor vorzugsweise im Bereich zwischen 0,3 bis 3 bar absolut, besonders bevorzugt im Bereich von 0,8 bis 3,0 bar absolut. Die Temperatur liegt dabei vorzugsweise im Bereich von 250 bis 550°C, insbesondere im Bereich von 300 bis 500°C.

Um die Reaktion in der Gasphase durchführen zu können, ist es weiterhin bevorzugt, das Amin und das Phosgen gasförmig zuzugeben. Hierzu weist das Amin vorzugsweise eine Temperatur im Bereich von 200 bis 400°C auf. Der Druck des zugegebenen Amins liegt dabei vorzugsweise im Bereich zwischen 0,05 bis 3 bar absolut. Die Temperatur des zugegebenen Phosgens liegt vorzugsweise im Bereich von 250 bis 450°C. Hierzu wird das Phosgen üblicherweise vor Zugabe auf dem Fachmann bekannte Weise erwärmt.

Zur Erwärmung des Phosgens und des Amins und zur Verdampfung des Amins wird zum Beispiel eine elektrische Beheizung oder eine direkte oder indirekte Aufheizung durch Verbrennung eines Brennstoffs eingesetzt. Als Brennstoffe werden üblicherweise Brenngase, beispielsweise Erdgas, eingesetzt. Durch die Absenkung der Siedetemperatur durch Absenkung des Drucks des Amins ist jedoch auch eine Beheizung, zum Beispiel durch Wasserdampf, möglich. In Abhängigkeit von der Siedetemperatur des Amins wird hierbei der Druck des Wasserdampfes ausgewählt. Ein geeigneter Dampfdruck des Wasserdampfes liegt zum Beispiel im Bereich von 40 bis 100 bar. Daraus ergibt sich eine Temperatur des Wasserdampfes im Bereich von 250 bis 311°C.

Im Allgemeinen ist es erforderlich, das Amin mehrstufig auf die Reaktionstemperatur zu erwärmen. Im Allgemeinen wird das Amin hierzu zunächst vorgewärmt, dann verdampft und anschließend überhitzt. Im Allgemeinen erfordert die Verdampfung die längsten Verweilzeiten und führt so zu Zersetzungen des Amins. Um dies zu minimieren, ist eine Verdampfung bei niedrigeren Temperaturen, wie es sich zum Beispiel durch den niedrigeren Druck ergibt, vorteilhaft. Um nach der Verdampfung das verdampfte Amin auf Reaktionstemperatur zu überhitzen, ist im Allgemeinen eine Beheizung mit Wasserdampf nicht ausreichend. Zur Überhitzung wird daher üblicherweise eine elektrische Beheizung oder eine direkte oder indirekte Aufheizung durch Verbrennung eines Brennstoffs eingesetzt.

Im Unterschied zur Verdampfung des Amins erfolgt die Verdampfung des Phosgens im Allgemeinen bei deutlich niedrigeren Temperaturen. Aus diesem Grund kann zur Verdampfung des Phosgens im Allgemeinen Wasserdampf eingesetzt werden. Alternativ kann die Verdampfung des Phosgens auch durch Wärmeintregration erfolgen, indem zum Beispiel die im Quench gewonnene Wärme zur Verdampfung des Phosgens genutzt wird. Hierdurch kann die Verdampfung gesamtenergieneutral erfolgen. Neben der im Quench gewonnenen Wärme kann auch jeder beliebige andere Stoffstrom, der eine höhere Temperatur aufweist als die Verdampfungstemperatur des Phosgens, genutzt werden. Dies sind zum Beispiel bei einer sich an den Quench anschließenden Kondensation anfallende Kondensatströme. Jedoch ist auch die notwendige Überhitzung des Phosgens, um dieses auf Reaktionstemperatur zu erwärmen, im Allgemeinen nur durch eine elektrische Beheizung oder eine direkte oder indirekte Aufheizung durch Verbrennung eines Brennstoffes möglich.

Der Reaktor, der zur Phosgenierung des Amins zur Herstellung von Isocyanaten eingesetzt wird, ist dem Fachmann bekannt. Im Allgemeinen werden als Reaktor Rohrreaktoren eingesetzt. Im Reaktor wird das Amin mit dem Phosgen zum korrespondierenden Isocyanat und Chlorwasserstoff umgesetzt. Üblicherweise wird das Phosgen in Überschuss zugegeben, so dass das im Reaktor entstehende Reaktionsgas neben dem gebildeten Isocyanat und dem Chlorwasserstoff auch Phosgen enthält.

Amine, die zur Herstellung von Isocyanaten eingesetzt werden können, sind Monoamine, Diamine, Triamine oder höherwertige Amine. Bevorzugt werden Monoamine oder Diamine eingesetzt. Entsprechend des eingesetzten Amins ergeben sich die korrespondierenden Monoisocyanate, Diisocyanate, Triisocyanate oder höherwertigen Isocyanate. Bevorzugt werden Monoisocyanate oder Diisocyanate mit dem erfindungsgemäßen Verfahren hergestellt.

Diamine und Diisocyanate können aliphatisch, cycloaliphatisch oder aromatisch sein.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

Im Folgenden wird die Bezeichnung (cyclo)aliphatische Isocyanate für cycloaliphatische und/oder aliphatische Isocyanate verwendet.

Beispiele für aromatische Mono- und Diisocyanate sind bevorzugt solche mit 6 bis 20 C-Atomen, beispielsweise Phenylisocyanat, monomeres 2,4'- und/oder 4,4'-Methylendi(phenylisocyanat) (MDI), 2,4- und/oder 2,6-Toluylendiisocyanat (TDI) und 1,5- oder 1,8-Naphthyldiisocyanat (NDI).

Beispiele für (cyclo)aliphatische Diisocyanate sind aliphatische Diisocyanate wie 1,4-Tetramethylendiisocyanat, 1,6-Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, 1,5-Diisocyanatopentan, Neopentandiisocyanat, Derivate des Lysindiisocyanats, Tetramethylxylylendiisocyanat (TMXDI), Trimethylhexandiisocyanat oder Tetramethylhexandiisocyanat, sowie 3(beziehungsweise 4)-8(beziehungsweise 9)-Bis(isocyanatomethyl)-tricyclo-[5.2.1.0^{2.6}]-decan-Isomerengemische, sowie cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5-trimethyl-5-(isocyanatomethyl)cyclohexan (Isoforondiisocyanat), 1,3- oder 1,4-Bis(isocyanatomethyl)cyclohexan, 2,4- oder 2,6-Diisocyanato-1-methylcyclohexan.

Bevorzugte (cyclo)aliphatische Diisocyanate sind 1,6-Diisocyanatohexan, 1-Isocyanato-3,3,5-trimethyl-5-(isocyanato-methyl)cyclohexan und 4,4'-Di(isocyanatocyclo-hexyl)methan. Besonders bevorzugt sind 1,6-Diisocyanatohexan, 1-Isocyanato-3,3,5-trimethyl-5-(isocyanatomethyl)cyclohexan, 1,5-Diisocyanatopentan und 4,4'-Di(isocyanatocyclohexyl)methan.

Beispiele für aromatische Diisocyanate sind 2,4-, 2,6-Toluylendiisocyanat, Methylendiphenylisocyanat oder Isomerengemische davon.

Amine, die bei dem erfindungsgemäßen Verfahren zur Reaktion zu den korrespondierenden Isocyanaten eingesetzt werden, sind solche, bei denen das Amin, die korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 Mol-%, besonders bevorzugt zu höchstens 1 Mol-% und ganz besonders bevorzugt zu höchstens 0,5 Mol-% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1,5-Diaminopentan, 1,3-Bis(aminomethyl)cyclohexan, 1-Amino-3,3,5-trimethyl-5-amino-methylcyclohexan (IPDA) und 4,4-Diaminodicyclohexylmethan. Bevorzugt verwendet werden 1,6-Diaminohexan (HDA) und 1,5-Diaminopentan.

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (Mol/Mol)-Gemisch, Diaminobenzol, 2,6-Xylidin, Naphthyldiamin (NDA) und 2,4'- oder 4,4'-Methylen(diphenyldiamin) (MDA) oder Isomerengemische davon. Bevorzugt sind unter diesen die Diamine, besonders bevorzugt sind 2,4- und/oder 2,6-TDA oder 2,4'- und/oder 4,4'-MDA.

Zur Herstellung von Monoisocyanaten können ebenfalls aliphatische, cycloaliphatische oder aromatische Amine, üblicherweise Monoamine, eingesetzt werden. Als aromatisches Monoamin ist insbesondere Anilin bevorzugt.

Bei der Gasphasenphosgenierung ist es anzustreben, dass die im Reaktionsverlauf auftretenden Verbindungen, also Edukte (Amin und Phosgen), Zwischenprodukte (insbesondere die intermediär entstehenden Mono- und Dicarbamoylchloride), Endprodukte (Isocyanat), sowie gegebenenfalls zudosierte inerte Verbindungen, unter den Reaktionsbedingungen in der Gasphase verbleiben. Sollten diese oder andere Komponenten sich aus der Gasphase zum Beispiel an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für Auftreten der Aminhydrochloride, die sich aus freien Aminogruppen und Chlorwasserstoff bilden, da die resultierenden Aminhydrochloride leicht ausfallen und nur schwer wieder verdampfbar sind.

Neben dem Einsatz eines Rohrreaktors ist es auch möglich, im Wesentlichen quaderförmige Reaktionsräume, beispielsweise Plattenreaktoren zu verwenden. Auch jeder beliebige andere Querschnitt des Reaktors ist möglich.

Um die Bildung von Nebenprodukten zu vermeiden, ist es bevorzugt, Phosgen im Überschuss zuzuführen. Um nur den für die Reaktion notwendigen Anteil an Aminen zuzuführen, ist es möglich, das Amin mit einem Inertgas zu mischen. Durch den Anteil an Inertgas im Amin lässt sich die Menge des zugeführten Amins bei vorgegebener Geometrie der Zufuhröffnungen für das Amin und das Phosgen einstellen. Inertmedien, die zugegeben werden können, sind solche, die im Reaktionsraum gasförmig vorliegen und nicht mit den im Reaktionsverlauf auftretenden Verbindungen reagieren. Als Inert-medium können zum Beispiel Stickstoff, Edelgase wie Helium oder Argon, Aromaten wie Chlorbenzol, o-Dichlorbenzol, Trichlorbenzol, Toluol, Xylol, Chlornaphtalin, Decahydronaphtalin, Kohlenstoffdioxid oder Kohlenstoffmonoxid eingesetzt werden. Bevorzugt werden jedoch Stickstoff und/oder Chlorbenzol als Inertmedium verwendet.

Alternativ ist es jedoch auch möglich, zum Beispiel um einen zu großen Überschuss an Phosgen zu vermeiden, das Inertmedium dem Phosgen zuzumischen.

Im Allgemeinen wird das Inertmedium in einer Menge zugesetzt, dass das Verhältnis der Gasvolumina von Inertmedium zu Amin beziehungsweise zu Phosgen weniger als 0,0001 bis 30, bevorzugt weniger als 0,01 bis 15 und besonders bevorzugt weniger als 0,1 bis 5 beträgt.

Um die Bildung unerwünschter Nebenprodukte zu reduzieren beziehungsweise zu vermeiden und weiterhin auch eine Zersetzung des gebildeten Isocyanats zu unterbinden, wird das Reaktionsgas unmittelbar nach der Reaktion in einem Quench abgekühlt. Hierzu wird ein vorzugsweise flüssiges Quenchmedium zugegeben. Durch Erwärmung oder Verdampfung des Quenchmediums nimmt dieses Wärme auf und führt zu einer schnellen Abkühlung des Reaktionsgases.

Erfindungsgemäß enthält das Quenchmedium zumindest einen Teil des den Quench verlassenden Stoffgemischs. Dieser enthält im Allgemeinen gegebenenfalls eingesetztes Lösungsmittel, bei der Reaktion gebildetes Isocyanat, sowie gegebenenfalls Reste an Phosgen und HCl.

Um zu vermeiden, dass sich in Rohrleitungen, Regeleinrichtungen und sonstigen Apparateteilen, insbesondere in den Zerstäuberdüsen des Quenchs, Ablagerungen bilden, werden gegebenenfalls im Quenchmedium enthaltene Feststoffpartikel vor Zugabe in den Quench entfernt.

In einer ersten Ausführungsform werden die gegebenenfalls in dem Quenchmedium enthaltenen Feststoffpartikel in einem Hydrozyklon oder einem Filter aus dem Quenchmedium entfernt. Der Filter oder der Hydrozyklon kann dabei an jeder beliebigen Stelle vor den Zerstäuberdüsen positioniert sein.

Wenn zur Abtrennung der gegebenenfalls im Quenchmedium enthaltenen Feststoffpartikel ein Filter eingesetzt wird, so eignet sich jeder beliebige, dem Fachmann bekannte Filter. Geeignete Filter sind zum Beispiel kontinuierlich wie auch diskontinuierlich arbeitende Apparate wie Oberflächen- oder Tiefenfilter, beispielsweise Siebfilter, Nutschenfilter, Kerzenfilter, Blattfilter, Tiefenfilter, Kammerfilter und Membranfilter. Diese Filter können über Druck (Druckfilter) oder über Unterdruck (Saugfilter) betrieben werden.

Neben Filtern können auch Zentrifugen, beispielsweise Schälzentrifugen, Tellerzentrifugen, Siebzentrifugen oder Schubzentrifugen oder Schwerkraftabscheider zum Abtrennen von partikulären Bestandteilen eingesetzt werden.

In einer alternativen Ausführungsform werden die gegebenenfalls in dem Quenchmedium enthaltenen Feststoffpartikel durch Verdampfung und Rekondensation aus dem Quenchmedium entfernt. Durch die Verdampfung gehen flüssige Bestandteile des Quenchmediums in die Dampfphase über und werden anschließend wieder auskondensiert. Feststoffpartikel und schwersiedende Bestandteile werden auf diese Weise abgetrennt. Die Verdampfung und Rekondensation kann zum Beispiel in einer Destillationskolonne durchgeführt werden. Alternativ ist es auch möglich, einen beliebigen Verdampfer zum Verdampfen einzusetzen und diesem einen Kondensator nachzuschalten.

Um eine schnelle Abkühlung des Reaktionsgases im Quench zu erzielen, wird das Quenchmedium im Allgemeinen flüssig zugegeben. Die Temperatur des Quenchmediums liegt dabei vorzugsweise im Bereich von 0 bis 250 °C, insbesondere im Bereich von 20 bis 220 °C. Durch das Eindüsen des Quenchmediums in das heiße Reaktionsgas wird das Quenchmedium erwärmt und/oder verdampft. Die für das Erwärmen und die Verdampfung des Quenchmediums notwendige Wärme wird dem Reaktionsgas entzogen und das Reaktionsgas wird auf diese Weise abgekühlt. Die Temperatur, auf die das Reaktionsgas abgekühlt wird, lässt sich zum Beispiel durch die Menge und die Temperatur des zugegebenen Quenchmediums einstellen.

Um die Temperatur, mit der das Quenchmedium in den Quench zugegeben wird, einzustellen, wird das Quenchmedium vorzugsweise durch einen Wärmetauscher geleitet. Je nach Eintrittstemperatur des Quenchmediums in den Wärmetauscher kann das Quenchmedium in diesem erwärmt oder abgekühlt werden. Ein Abkühlen ist zum Beispiel dann erforderlich, wenn der Teil des Produktstromes, der als Quenchmedium eingesetzt wird, direkt im Anschluss an den Quench entnommen wird. Ein Erwärmen kann sich zum Beispiel dann ergeben, wenn der Teil des Produktstromes, der als Quenchmedium eingesetzt wird, am Ende der Aufbereitungsstrecke entnommen wird und eine Temperatur aufweist, die niedriger ist als die gewünschte Temperatur, mit der das Quenchmedium in den Quench zugegeben werden soll. Im Allgemeinen wird es jedoch erforderlich sein, das Quenchmedium vor Zugabe in den Quench abzukühlen.

Um Lösungsmittelverluste im Quenchmedium auszugleichen, ist es bevorzugt, dem Quenchmedium vor Zugabe in den Quench Lösungsmittel zuzugeben. Geeignete Lösungsmittel, die im Quenchmedium enthalten sind, sind zum Beispiel gegebenenfalls mit Halogen substituierte Kohlenwasserstoffe. Vorzugsweise ist das Lösungsmittel, das im Quenchmedium enthalten ist, ausgewählt aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophtalat, Tetrahydrofuran, Dimethylformamid, Xylol, Chlornaphthalin, Decahydronaphthalin und Toluol.

Der Anteil an Isocyanat im Quenchmedium kann im Bereich von 0 bis 100 % liegen. So liegt der Anteil an Isocyanat bei einem reinen Leichtsiederquench im Allgemeinen bei 0 % und bei einem reinen Hochsiederquench bei 100 %. Als Leichtsiederquench wird ein mit im Wesentlichen reinem Lösungsmittel als Quenchmedium betriebener Quench verstanden und als Hochsiederquench ein mit im Wesentlichen reinen Isocyanat betriebener Quench. Je nach Abzweigung des Quenchmediums im Prozess ist aber auch jede beliebige Zusammensetzung, die Lösungsmittel und Isocyanat enthält, möglich.

Zur weiteren Behandlung des Produktstromes schließen sich an den Quench in einer weiteren Ausführungsform weitere Stufen zur Abkühlung des Reaktionsgases an. In den einzelnen Stufen zur Abkühlung erfolgt dabei jeweils eine weitere Abkühlung des Produktstromes bis zum Erreichen der gewünschten Endtemperatur, mit der der Produktstrom beispielsweise einer nachfolgenden Aufarbeitung zugeführt wird.

Die weiteren Stufen zur Abkühlung, die sich an den Quench anschließen können, können zum Beispiel weitere Quenche oder Kondensatoren oder beliebige andere Stufen zur Abkühlung, die dem Fachmann bekannt sind, sein. Bevorzugt ist mindestens eine der sich an den Quench anschließenden Stufen zur Abkühlung des Produktstromes ein Kondensator. Als Kondensator eignet sich dabei jede beliebige, dem Fachmann bekannte Kondensatorbauart. Üblicherweise wird als Kondensator ein Wärmetauscher eingesetzt, der von einem Kühlmedium durchströmt wird. Als Kühlmittel kann zum Beispiel Wasser oder gekühltes Lösungsmittel eingesetzt werden. In diesem Fall kondensiert das Gas zumindest teilweise an den Wandungen des Kondensators aus. Die so entstehende Flüssigkeit läuft ab und wird gesammelt und dem Kondensator entnommen.

Nach dem Kondensieren des Produktstromes wird im Allgemeinen eine Aufbereitung angeschlossen. So ist es zum Beispiel möglich, dass das auskondensierte Gemisch in einem Lösungsmittel gewaschen wird. Als Lösungsmittel können zum Beispiel die gleichen Stoffe eingesetzt werden, die auch als Quenchmedium eingesetzt werden können.

Bei der Wäsche wird das Isocyanat selektiv in die Waschlösung überführt. Anschließend wird, bevorzugt durch Rektifikation, das erhaltene Gemisch in Isocyanat, Lösungsmittel, Phosgen und Chlorwasserstoff aufgetrennt.

Alternativ zur Abkühlung des Produktstromes ist es auch möglich, dass der Produktstrom nach Austritt aus dem Quench einer Trennstufe zugeführt wird. Eine entsprechende Trennstufe kann sich alternativ jedoch auch zum Beispiel dem Kondensator anschließen. Bevorzugt schließt sich jedoch die Trennstufe direkt an den Quench an. Als Trennstufen eignen sich zum Beispiel Destillationskolonnen oder Wäscher.

Wenn die Trennstufe ein Wäscher ist, so wird der den Quench verlassende Produktstrom vorzugsweise - wie zuvor beschrieben - mit einem Lösungsmittel gewaschen. Hierbei wird das Isocyanat selektiv in die Waschlösung überführt. An die Wäsche schließt sich dann eine Trennung, bevorzugt mittels Rektifikation, an.

Wenn die Trennstufe eine Destillationskolonne ist, so wird der gasförmige Produktstrom der Rektifikationskolonne zugeführt. Die Rektifikationskolonne wird vorzugsweise so betrieben, dass die Temperatur am Kopf der Rektifikationskolonne niedriger ist als die Siedetemperatur des Produktstromes. Auf diese Weise kondensieren in der Destillationskolonne selektiv einzelne Bestandteile des Produktstromes aus und können der Kolonne am Sumpf, über Kopf und gegebenenfalls über Seitenabzüge entnommen werden.

Wenn die Trennstufe ein Wäscher ist, so eignet sich insbesondere ein Waschturm, in dem aus dem gasförmigen Produktstrom das gebildete Isocyanat durch Kondensation in einem inerten Lösungsmittel abgetrennt wird, während überschüssiges Phosgen, Chlorwasserstoff und gegebenenfalls das Inertmedium den Waschturm gasförmig durchlaufen. Bevorzugt wird dabei die Temperatur des inerten Lösungsmittels so gewählt, dass das zum Amin gehörige Carbamoylchlorid im gewählten Waschmedium gelöst vorliegt. Besonders bevorzugt wird dabei die Temperatur des inerten Lösungsmittels oberhalb der Schmelztemperatur des zum Amin gehörigen Carbamoylchlorids gehalten.

Als Wäscher eignet sich jeder beliebige, dem Fachmann bekannte Wäscher. So können zum Beispiel Rührbehälter oder andere herkömmliche Apparaturen, beispielsweise Kolonnen oder Mixer-Settler-Apparaturen eingesetzt werden.

Das Waschen und die Aufarbeitung des den Quench verlassenden Gemischs aus Reaktionsgas und Quenchmedium erfolgt dabei im Allgemeinen wie beispielsweise in WO-A 2007/028715 beschrieben.

Wenn zur Aufbereitung des Produktstromes ein Kondensator eingesetzt wird, ist es bevorzugt, das Quenchmedium dem Kondensator zu entnehmen. Bei einer Aufbereitung durch Rektifikation erfolgt vorzugsweise eine Abtrennung des als Quenchmedium eingesetzten Lösungsmittels. Das Lösungsmittel enthält in diesem Fall noch Anteil an Isocyanaten. Das so abgetrennte Gemisch aus Lösungsmittel und Isocyanat wird dann als Quenchmedium eingesetzt.

Wenn ein Teil des Produktstromes als Quenchmedium eingesetzt wird, so ist es möglich, diesen Teil zum Beispiel nach dem Abkühlen aus dem Produktstrom abzuzweigen. Alternativ kann der als Quenchmedium eingesetze Teil des Produktstromes auch nach einer sich an den Quench anschließenden Aufarbeitung aus dem Produktstrom abgezweigt werden.

Im Folgenden wird die Erfindung beispielhaft an einer Zeichnung näher beschrieben.

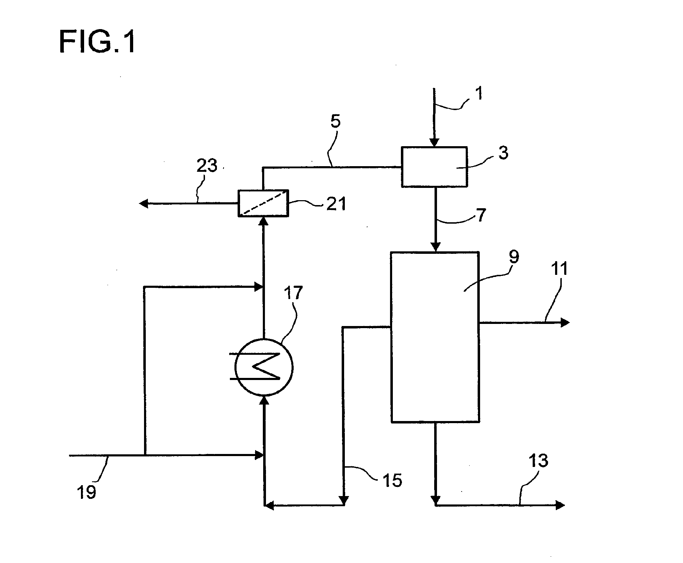

Die einzige Figur zeigt eine schematische Darstellung des erfindungsgemäßen Verfahrens.

Ein einen Reaktor zur Herstellung von Isocyanaten durch Umsetzung der korrespondierenden Amine mit Phosgen in der Gasphase verlassendes, Isocyanat und Chlorwasserstoff enthaltendes Reaktionsgas 1 wird einem Quench 3 zugeführt. Durch Zugabe eines Quenchmediums 5 in den Quench 3 wird das Reaktionsgas 1 unter Bildung eines Reaktionsgas und Quenchmedium enthaltenden Produktstromes 7 abgekühlt. Der Produktstrom 7 enthält im Allgemeinen eine flüssige und eine gasförmige Phase.

Der Produktstrom 7 wird in der hier dargestellten Ausführungsform einer Vorrichtung zur Phasentrennung 9 zugeführt. Als Phasentrenner 9 eignet sich jede beliebige, dem Fachmann bekannte Vorrichtung, in der eine flüssige Phase von einer gasförmigen Phase getrennt werden kann.

Der Vorrichtung zur Phasentrennung 9 wird eine Gasphase 11 und eine Flüssigphase 13 entnommen. Die Gasphase enthält im Allgemeinen Phosgen, HCl, gegebenenfalls Inertmedium und Lösungsmittel, und die Flüssigphase 13 enthält im Allgemeinen das Isocyanat, schwersiedende Nebenprodukte und gegebenenfalls Lösungsmittel.

Als weiterer Strom wird der Vorrichtung zur Phasentrennung 9 in der hier dargestellten Ausführungsform auch Quenchmedium 15 entnommen. Das Quenchmedium 15 kann dabei gegebenenfalls Feststoffpartikel enthalten. Im Allgemeinen enthält das Quenchmedium 15 Lösungsmittel und Isocyanat. Das Quenchmedium 15 wird einem Wärmetauscher 17 zugeführt, in dem dieses auf die gewünschte Zufuhrtemperatur in den Quench 3 abgekühlt wird. Gegebenenfalls mit der Gasphase 11 oder der Flüssigphase 13 ausgeschleustes Lösungsmittel wird über eine Lösungsmittelzufuhr 19 ergänzt. Das Lösungsmittel 19 kann dabei alternativ vor dem Wärmetauscher 17 oder nach dem Wärmetauscher 17 zugeführt werden. Auch ist es möglich, den Lösungsmittelstrom aufzuspalten und einen Teil des Lösungsmittels vor dem Wärmetauscher 17 und einen Teil des Lösungsmittels nach dem Wärmetauscher 17 zuzuführen. Je nach Temperatur des Lösungsmittels erfolgt durch die Zugabe des Lösungsmittels 19 eine weitere Abkühlung des Quenchmediums 15. Die Lösungsmittelzufuhr 19 kann dabei aus einer beliebigen Verfahrensstufe gespeist werden, oder es wird Lösungsmittel von außerhalb zugegeben.

An den Wärmetauscher 17 schließt sich mindestens ein Partikelabscheider 21 an. Als Partikelabscheider 21 kann zum Beispiel ein Hydrozyklon oder ein Filter eingesetzt werden. Alternativ ist es auch möglich, als Partikelabscheider zum Beispiel eine Destillationskolonne oder einen Verdampfer und einen Kondensator einzusetzen. Im Partikelabscheider 21 werden gegebenenfalls im Quenchmedium 5 enthaltene Feststoffpartikel entfernt. In Abhängigkeit vom eingesetzten Partikelabscheider 21 können die Feststoffpartikel zum Beispiel als Schlamm, als Filterkuchen oder auch als Sumpf einer Destillation entnommen werden. Der Feststoff enthaltende Strom ist mit Bezugszeichen 23 bezeichnet.

Wenn mehrere Partikelabscheider 21 eingesetzt werden, so können mehrere gleichartige Partikelabscheider oder unterschiedliche Partikelabscheider in Reihe oder parallel eingesetzt werden. So ist es zum Beispiel möglich, mehrere Filter, mehrere Hydrozyklone oder auch Filter und Hydrozyklone einzusetzen. Wenn mehrere Filter als Partikelabscheider 21 eingesetzt werden, so ist es zum Beispiel möglich, mehrere Filter mit unterschiedlicher Porengröße einzusetzen, wobei die Porengröße in Strömungsrichtung des Quenchmediums von Filter zu Filter abnimmt. Es kann aber auch vorteilhaft sein, mehrere gleichartige Partikel-Abscheider parallel einzusetzen und wechselseitig zu regenerieren. Das so von Feststoffpartikeln gereinigte Quenchmedium wird dann dem Quench 3 zur Abkühlung des Reaktionsgases 1 zugeführt.

Neben der hier dargestellten Ausführungsform, bei der das Quenchmedium 15 der Vorrichtung zur Phasentrennung 9 entnommen wird, ist es alternativ auch möglich, direkt einen Teil des Produktstroms 7 nach dem Verlassen des Quenches 3 abzuzweigen. Auch ist es möglich, einen Teil der Flüssigphase 13, die der Vorrichtung zur Phasentrennung 9 entnommen wird, als Quenchmedium abzuzweigen. Es ist aber auch möglich den Quench nur über die Lösungsmittelzufuhr 19 zu speisen. In diesem Fall verlässt alles flüssige Produkt die Vorrichtung zur Phasentrennung als Flüssigphase 13.

Alternativ ist es weiterhin möglich, anstelle einer Vorrichtung zur Phasentrennung andere Stufen zur Aufarbeitung einzusetzen. In diesem Fall ist es ebenfalls möglich an jeder beliebigen Position einen Teil des Produktstromes zu entnehmen, um diesen nach entsprechender Aufarbeitung zum Beispiel durch Kondensation und Partikelabscheidung als Quenchmedium 5 einzusetzen.

### Bezugszeichenliste

- 1: Reaktionsgas
- 3: Quench
- 5: Quenchmedium
- 7: Produktstrom
- 9: Vorrichtung zur Phasentrennung
- 11: Gasphase
- 13: Flüssigphase
- 15: Quenchmedium
- 17: Wärmetauscher
- 19: Lösungsmittel
- 21: Partikelabscheider
- 23: Feststoff enthaltender Strom

## Patentansprüche

1. Verfahren zur Herstellung von Isocyanaten durch Umsetzung der korrespondierenden Amine mit Phosgen in der Gasphase, gegebenenfalls in Gegenwart eines Inertmediums, bei dem das Amin und das Phosgen zunächst gemischt werden und in einem Reaktor zum Isocyanat umgesetzt werden, wobei ein den Reaktor verlassendes, Isocyanat und Chlorwasserstoff enthaltendes Reaktionsgas (1) in einem Quench (3) durch Zugabe eines flüssigen Quenchmediums (5) abgekühlt wird, wobei ein Gemisch aus Reaktionsgas und Quenchmedium als Produktstrom (7) entsteht, **dadurch gekennzeichnet, dass** als Quenchmedium (5) ein Gemisch eingesetzt wird, das mindestens ein Lösungsmittel oder Isocyanat enthält und das einem der Reaktion nachgeschalteten Aufarbeitungsverfahren entnommen wird, wobei in dem Quenchmedium (5) gegebenenfalls enthaltene Feststoffpartikel vor Zugabe in den Quench (3) entfernt werden.

2. Verfahren gemäß Anspruch 1, **dadurch gekennzeichnet, dass** die gegebenenfalls in dem Quenchmedium (5) enthaltenen Feststoffpartikel in einem Hydrozyklon, einem Filter, einer Zentrifuge oder einem Schwerkraftabscheider (21) aus dem Quenchmedium entfernt werden.

3. Verfahren gemäß Anspruch 2, **dadurch gekennzeichnet, dass** der Filter ein Siebfilter, Nutschenfilter, Kerzenfilter, Blattfilter, Tiefenfilter, Kammerfilter oder Membranfilter ist.

4. Verfahren gemäß Anspruch 1, **dadurch gekennzeichnet, dass** die gegebenenfalls in dem Quenchmedium (5) enthaltenen Feststoffpartikel durch Verdampfung und Rekondensation aus dem Quenchmedium entfernt werden.

5. Verfahren gemäß einem der Ansprüche 1 bis 4, **dadurch gekennzeichnet, dass** das Quenchmedium (5) als Lösungsmittel einen gegebenenfalls mit Halogen substituierten Kohlenwasserstoff enthält.

6. Verfahren gemäß einem der Ansprüche 1 bis 5, **dadurch gekennzeichnet, dass** das Quenchmedium (5) ein Lösungsmittel enthält, das ausgewählt ist aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophthalat, Tetrahydrofuran, Dimethylformamid, Xylol, Chlornaphthalin, Decahydronaphthalin und Toluol.

7. Verfahren gemäß einem der Ansprüche 1 bis 6, **dadurch gekennzeichnet, dass** das Quenchmedium (5) zumindest einen Teil des Produktstromes (7) enthält.

8. Verfahren gemäß einem der Ansprüche 1 bis 7, **dadurch gekennzeichnet, dass** sich an den Quench (3) weitere Stufen zur Abkühlung des Reaktionsgases anschließen.

9. Verfahren gemäß Anspruch 8, **dadurch gekennzeichnet, dass** mindestens eine der sich an den Quench (3) anschließenden Stufen zur Abkühlung des Reaktionsgases ein Kondensator ist.

10. Verfahren gemäß einem der Ansprüche 1 bis 9, **dadurch gekennzeichnet, dass** der als Quenchmedium (5) eingesetzte Teil des Produktstroms (7) nach einer sich an den Quench (3) anschließende Aufarbeitung aus dem Produktstrom abgezweigt wird.

11. Verfahren gemäß Anspruch 10, **dadurch gekennzeichnet, dass** die sich an den Quench (3) anschließende Aufarbeitung eine Trennstufe umfasst.

12. Verfahren gemäß Anspruch 11, **dadurch gekennzeichnet, dass** die Trennstufe eine Destillationskolonne oder ein Wäscher ist.

## Claims

1. A process for preparing isocyanates by reacting the corresponding amines with phosgene in the gas phase, optionally in the presence of an inert medium, in which the amine and the phosgene are first mixed and converted to the isocyanate in a reactor, and in which a reaction gas (1) which comprises isocyanate and hydrogen chloride leaving the reactor is cooled in a quench (3) by adding a liquid quench medium (5) to form a mixture of reaction gas and quench medium as the product stream (7) which comprises using, as the quench medium (5) a mixture which comprises at least one solvent or isocyanate and which is withdrawn from a workup process connected downstream of the reaction, any solid particles present in the quench medium (5) being removed before addition to the quench (3).

2. The process according to claim 1, wherein the solid particles which may be present in the quench medium (5) are removed from the quench medium in a hydrocyclone, a filter, a centrifuge or a gravitational separator (21).

3. The process according to claim 2, wherein the filter is a screen filter, suction filter, candle filter, leaf filter, depth filter, chamber filter or membrane filter.

4. The process according to claim 1, wherein the solid particles which may be present in the quench medium (5) are removed from the quench medium by evaporation and recondensation.

5. The process according to any of claims 1 to 4, wherein the quench medium (5) comprises, as a solvent, an optionally halogen-substituted hydrocarbon.

6. The process according to any of claims 1 to 5, wherein the quench medium (5) comprises a solvent which is selected from the group consisting of monochlorobenzene, dichlorobenzene, trichlorobenzene, hexane, benzene, 1,3,5-trimethylbenzene, nitrobenzene, anisole, chlorotoluene, o-dichlorobenzene, diethyl isophthalate, tetrahydrofuran, dimethylformamide, xylene, chloronaphthalene, decahydronaphthalene and toluene.

7. The process according to any of claims 1 to 6, wherein the quench medium (5) comprises at least a portion of the product stream (7).

8. The process according to any of claims 1 to 7, wherein the quench (3) is followed by further stages for cooling the reaction gas.

9. The process according to claim 8, wherein at least one of the stages for cooling the reaction gas which follows the quench (3) is a condenser.

10. The process according to any of claims 1 to 9, wherein the portion of the product stream (7) used as the quench medium (5) is branched off out of the product stream after a workup which follows the quench (3).

11. The process according to claim 10, wherein the workup which follows the quench (3) comprises a separating stage.

12. The process according to claim 11, wherein the separating stage is a distillation column or a scrubber.

## Revendications

1. Procédé pour la production d'isocyanates par mise en réaction des amines correspondantes avec du phosgène dans la phase gazeuse, éventuellement en présence d'un milieu inerte, dans lequel d'abord on mélange l'amine et le phosgène, puis on les convertit en l'isocyanate dans un réacteur, en refroidissant un gaz de réaction (1) contenant de l'isocyanate et du chlorure d'hydrogène, quittant le réacteur, dans un refroidisseur (3) par addition d'un milieu liquide de refroidissement (5), de sorte qu'il se forme un mélange de gaz de réaction et de milieu de refroidissement en tant que courant de produit (7), **caractérisé en ce qu'**on utilise comme milieu de refroidissement (5) un mélange qui contient au moins un solvant ou un isocyanate et qui est prélevé d'un processus de traitement final raccordé en aval à la réaction, des particules de matière solide éventuellement contenues dans le milieu de refroidissement (5) étant éliminées avant l'introduction dans le refroidisseur (3).

2. Procédé selon la revendication 1, **caractérisé en ce que** les particules de matière solide éventuellement contenues dans le milieu de refroidissement (5) sont éliminées du milieu de refroidissement dans un hydrocyclone, un filtre, une centrifugeuse ou un séparateur par gravité (21).

3. Procédé selon la revendication 2, **caractérisé en ce que** le filtre est un filtre à tamis, un filtre Nutsche, un filtre à bougies, un filtre à plaques, un filtre à lit profond, un filtre à cellules ou un filtre à membrane.

4. Procédé selon la revendication 1, **caractérisé en ce que** les particules de matière solide éventuellement contenues dans le milieu de refroidissement (5) sont éliminées du milieu de refroidissement par évaporation et recondensation.

5. Procédé selon l'une quelconque des revendications 1 à 4, caractérisé en ce le milieu de refroidissement (5) contient en tant que solvant un hydrocarbure éventuellement substitué par halogène.

6. Procédé selon l'une quelconque des revendications 1 à 5, **caractérisé en ce que** le milieu de refroidissement (5) contient un solvant qui est choisi dans le groupe constitué par le monochlorobenzène, le dichlorobenzène, le trichlorobenzène, l'hexane, le benzène, le 1,3,5-triméthylbenzène, le nitrobenzène, l'anisole, le chlorotoluène, l'o-dichlorobenzène, l'isophtalate de diéthyle, le tétrahydrofurane, le diméthylformamide, le xylène, le chloronaphtalène, le décahydronaphtalène et le toluène.

7. Procédé selon l'une quelconque des revendications 1 à 6, **caractérisé en ce que** le milieu de refroidissement (5) contient au moins une partie du courant de produit (7).

8. Procédé selon l'une quelconque des revendications 1 à 7, **caractérisé en ce qu'**au refroidisseur (3) font suite d'autres étages destinés au refroidissement du gaz de réaction.

9. Procédé selon la revendication 8, **caractérisé en ce qu'**au moins l'un des étages destinés au refroidissement du gaz de réaction, faisant suite au refroidisseur (3), est un condenseur.

10. Procédé selon l'une quelconque des revendications 1 à 9, **caractérisé en ce qu'**après un traitement final faisant suite au refroidisseur (3) la partie du courant de produit (7) qui est utilisée comme milieu de refroidissement (5) est détournée du courant de produit.

11. Procédé selon la revendication 10, **caractérisé en ce que** le traitement final faisant suite au refroidisseur (3) comprend un étage de séparation.

12. Procédé selon la revendication 11, **caractérisé en ce que** l'étage de séparation est une colonne de distillation ou un laveur.
